# EUROPEAN PATENT SPECIFICATION

(11) **EP 1 942 979 B1**
(45) Date of publication and mention of the grant of the patent: **19.04.2017**
(21) Application number: 06815129.9
(22) Date of filing: 21.09.2006
(51) Int. Cl.: A61M 31/00, A61M 16/04, A61M 25/10, A61M 29/02

(54) **AIRWAY BALLOON DILATOR**
ATEMWEGSBALLONDILATATOR
DISPOSITIF DE DILATATION A BALLONNET POUR VOIES RESPIRATOIRES

(30) Priority: 21.09.2005 US 231457; 20.09.2006 US 533562
(43) Date of publication of application: 16.07.2008
(73) Proprietor: Rutter, Michael John, Cincinnati OH 45215 (US)
(72) Inventor: Rutter, Michael John, Cincinnati OH 45215 (US)
(74) Representative: Klunker, Hans-Friedrich
(86) International application number: PCT/US2006/036869
(87) International publication number: WO 2007/035888

(56) References cited:
- EP-A2- 0 261 831
- WO-A2-2007/035888
- US-A- 3 481 339
- US-A- 4 791 923
- US-A- 4 791 923
- US-A- 4 983 167
- US-A- 5 196 024
- US-A- 5 320 634
- US-A1- 2003 041 863
- US-A1- 2005 148 997

## Description

### FIELD OF THE INVENTION

The present invention relates to medical care for the larynx, trachea or bronchi to relieve a stenosis, i.e. , the invention relates to a device for performing dilation of the larynx, trachea or bronchi.

### BACKGROUND OF THE INVENTION

Management of stenosis of the trachea and bronchi, including laryngo-tracheal and subglottic stenosis, is one of the most challenging problems for the head and neck surgeon. Subglottic stenosis is a congenital or acquired narrowing of the subglottic airway. In the early twentieth century subglottic stenosis was rare, and most cases occurred in adults. In the 1960's the incidence of acquired subglottic stenosis began to dramatically increase in the neonatal population, most likely the result of increased survival of low-birth-weight infants and the increased use of intubation in this population. In addition, long term intubation has become an accepted alternative to tracheotomy, leading to more and more incidences of tracheal stenosis. Accordingly, the management of this condition has undergone a revolution, and reconstructive surgery efforts have been directed towards this population.

Most patients with stenosis of the airway are referred to and are treated at large academic centers by physicians specially trained in this area. There is a wide range of presentation of subglottic stenosis with similarities and differences in the pediatric age group compared to adults. If the stenosis is severe and congenital, the patient will show signs of airway distress at birth. More commonly, the pediatric patient with subglottic stenosis is a neonate in the intensive care unit who has failed extubation, usually multiple times. Occasionally-patients will present in clinic with a tracheotomy and the report of some airway obstruction. Infants with mild subglottic stenosis may present with recurrent croup-like illnesses and poor feeding. Adults usually have a history of prior intubation with symptoms of progressive shortness of breath and noisy breathing.

Airway balloon dilation has been shown to be a safe and effective palliative procedure for treatment of mild congenital and acquired stenosis of the trachea and bronchi. Dilation of luminal human anatomy to treat stenoses can be dated back to the 16th Century with esophageal "bougie" dilation. Specific medical applications of luminal balloon dilation range from alimentary canal and airway dilation to dilation of the vasculature. Airway dilation dates back over 100 years ago with the invention and subsequent use of the first beveled rigid bronchoscopes for stricture management. The use of balloons to dilate airway strictures emerged in the mid-1980's with reports describing more specific utility of this procedure exclusively and in combination with other treatment modalities for airway stenosis. It was not until the early 1990's that the first balloon dilation involving flexible bronchoscopy was described.

Airway balloon dilation can be used to quickly re-establish tracheal or bronchial luminal patency to restore airflow in a way that doesn't cause excessive trauma to the patient. According to Poiseuille's Law, an increase in a tube's radius (such as the trachea or bronchus) can increase airflow by a power of 4 (airflow = radius of the tube⁴). That is, very small increases in the luminal diameter of the airway can lead to large increases in airflow through the lungs. Literature has reported the use of balloon dilation for the treatment of benign strictures of the airway. Fibrotic strictures, such as those secondary to tuberculosis, long-term endotracheal or tracheostomy tube placement, berylliosis, Wegener's granulomatosis, or sarcoidosis have been shown to be treatable with airway balloon dilation therapy with general success. Additionally, balloon dilation has been useful in treating strictures secondary to major surgical interventions such as lung transplantation, sleeve resection, bronchial re-implantation, and lobectomy. For the purpose of treating strictures secondary to malignant obstruction, dilation therapy can be used alone or in combination with other techniques such as surgical resection, cryotherapy, laser therapy, and stent placement, depending on the desired outcome for the patient.

Treatment with airway dilation can involve the clinician inserting increasingly larger tubes into the airway (e.g. endotracheal tubes or cat-tail (bougie) dilators), which creates significant shear forces on the airway mucosa. Although safe when performed by a skilled clinician, such a procedure sometimes induces unwanted trauma to the airway in the form of deep lacerations and hemoptysis. Further, current dilation practices do not permit dilation of a tracheal stenosis that is distal to a narrowing of the proximal airway (i.e. a mild subglottic stenosis).

Current airway balloon dilation procedures are typically carried out using angioplasty balloons; however, several limitations to the use of angioplasty balloons become evident when used on the airway. For example, it may be difficult to adequately ventilate the patient during the dilation period, since the typical angioplasty balloon does not include a connection to an oxygen source. Further, the shape of the angioplasty balloon may predispose the balloon to slide out of place during dilation, or the balloon may be limited to the amount of pressure that can be applied before the balloon bursts. Also, the typical angioplasty balloon can usually stretch the airway lumen but not permanently dilate it. Other factors associated with failure of airway balloon dilation include previous attempts at endoscopic repair, circumferential scarring, and loss of cartilaginous support.
US 4,791,923 discloses a tracheal tube in which a cuff is mounted on an elongated tube. The cuff includes an inner inflatable balloon member and an outer inflatable balloon member. The inner inflatable member is disposed on the elongated tube in surrounding relationship and the outer inflatable member is disposed on the elongated tube and over the inner member in surrounding relationship. The cuff provides a low pressure seal between the tube and the trachea.
WO 2005/023153 A2 discloses devices, systems, and methods for delivering implants such as stents into both open and solid regions of the body. The systems allow the choice of stent size after the stent has been placed in the body. This is achieved by configuring the system with one or more inflatable balloons, adapted to expand the implant after placement in the body. In a multiple-balloon device, the balloons may be stacked radially adjacent each other, and yet independently expandable. A high pressure balloon may be chosen for the outer balloon, which may be able to operate up to 20 atmospheres. For proper placement within the body, the system may comprise a core guide member, the core guide member having an inner diameter in the range of about 0.075 to 0.5 mm (0.003 to 0.020 inches).
EP 0 261 831 A2 discloses concentric independently inflatable/deflatable multiple diameter balloon angioplasty catheter systems. Three different designs are discloses: A steerable guidewire type, a bypass sidehole type, and an axial torque type. In the axial torque type, the balloons are directly bonded to a tapered guidewire. In the steerable guidewire type, the balloons are bonded to a catheter shaft, the shaft having a central lumen which is large enough to accommodate a steerable guidewire. The bypass sidehole type is similar to the steerable guidewire type, but has holes in the catheter shaft walls such that blood may enter into the central lumen of the shaft, bypass the balloons, and exit the lumen of the shaft downstream of the inflated balloons.

In light of the foregoing, it would be advantageous to provide a balloon dilator for the airway of a patient that is able to allow ventilation of the patient during balloon inflation. It would also be helpful to provide an airway balloon dilator that can provide increased inflation pressures during balloon dilation of the airway without balloon rupture. Further, it would be beneficial to provide a balloon that will not slip out of place in the patient's airway during balloon inflation. Finally, it is desirable to provide an airway balloon dilator that is capable of controlled cutting of scar tissue.

### SUMMARY OF THE INVENTION

Subject matter of the present invention is an apparatus for performing an airway balloon dilation procedure as claimed in claim 1. Embodiments of the invention are claimed in the dependent claims.

The present invention provides an airway balloon dilator for use to quickly reestablish laryngeal, tracheal or bronchial luminal patency to restore airflow in a way that avoids excessive trauma to the patient.

The invention provides an apparatus for performing an airway balloon dilation procedure at the site of a stenosis in the airway of a patient, the apparatus comprising a central axis, a hollow core adapted to allow the patient to be ventilated therethrough by having a central ventilating lumen with an inner diameter from 2.0 mm to 4.0 mm, an inflatable outer balloon having an external surface, and at least one inflatable inner balloon, the apparatus being insertable into the airway of a patient for movement of the balloons therein between a deflated configuration and an inflated configuration, the at least one inner balloon configured to inflate inside the outer balloon yet separately from the outer balloon and having, in the inflated configuration, a smaller diameter than the outer balloon has in the inflated configuration. The balloons are adapted to be individually dilated to between 810.6 kPa (8 atmospheres) to 2026.5 kPa (20 atmospheres).

Described herein is also a method for performing an airway balloon dilation procedure at the site of a stenosis in the airway of a patient, the method comprising: (1) inserting an apparatus into the airway, the apparatus including a central axis, a hollow core adapted to allow the patient to be ventilated therethrough, an inflatable outer balloon having an external surface, and at least one inflatable inner balloon, the apparatus being insertable into the airway of a patient for movement of the balloons therein between a deflated configuration and an inflated configuration, the at least one inner balloon configured to inflate inside the outer balloon yet separately from the outer balloon; (2) advancing the apparatus within the airway until the outer balloon is across the stenosis; and (3) inflating the balloon to cause and allow the external surface of the balloon to expand upon and dilate the stenosis.

Further described herein is an apparatus for performing an airway balloon dilation procedure at the site of a stenosis in the airway of a patient, the apparatus comprising a central axis, a hollow core adapted to allow the patient to be ventilated therethrough, an inflatable, dumbbell-shaped outer balloon having an external surface, at least one inflatable inner balloon, a flexible support member mounted along the central axis of the apparatus and on the external surface of the outer balloon, the flexible support member being substantially compliant with the external surface of the outer balloon during movement therewith, and at least one microsurgical blade attached to the support member and adapted to form an effective cutting edge upon inflation of the outer balloon, the apparatus being insertable into the airway of a patient for movement of the balloons therein between a deflated configuration and an inflated configuration, the at least one inner balloon configured to inflate inside the outer balloon yet separately from the outer balloon, the dumbbell shape of the outer balloon adapted to hold the outer balloon in position over the stenosis, and the at least one blade adapted to form an effective cutting edge upon inflation of the outer balloon.

The nature and advantages of the present invention will be more fully appreciated from the following drawings, detailed description and claims.

### BRIEF DESCRIPTION OF THE DRAWINGS

The accompanying drawings illustrate embodiments of the invention and, together with a general description of the invention given above, and the detailed description given below, serve to explain the principles of the invention.
FIG. 1 is a perspective view of one embodiment of the balloon dilator of the present invention.
FIG. 2 is a perspective view of one embodiment of the balloon dilator in which the outer balloon has a dumbbell shape and multiple inner balloons.
FIG. 3 is a perspective view of a flexible support member having microsurgical blades, the support member adapted to fit over the outer balloon according to one aspect of the invention.

### DETAILED DESCRIPTION OF THE INVENTION

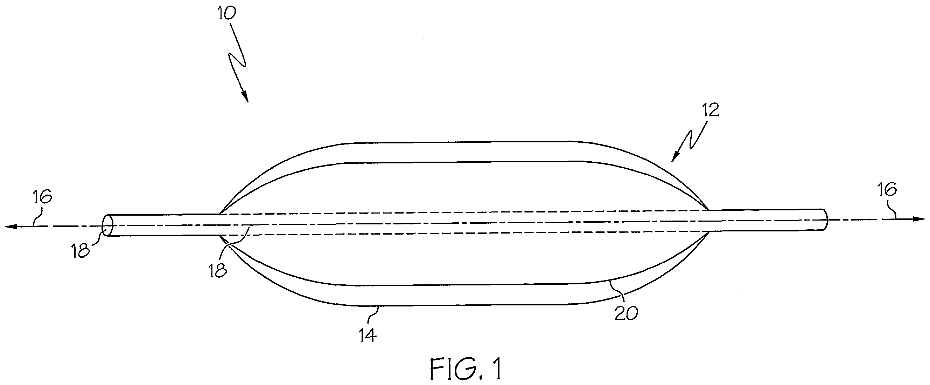

As illustrated in FIG. 1, one embodiment of the present invention is an apparatus 10 for performing an airway balloon dilation procedure at the site of a stenosis in the airway of a patient, the apparatus comprising an inflatable outer balloon 12 which has an external surface 14. The apparatus also comprises a central axis 16, a hollow core 18, and at least one inflatable inner balloon 20 adapted to inflate inside the outer balloon 12. The apparatus 10 is typically insertable into the airway of a patient for movement of the balloons 12, 20 between a deflated configuration and an inflated configuration. Further, the inner balloon 20 is designed to inflate inside the outer balloon 12 yet separately from the outer balloon, adding the ability of the apparatus to produce high dilation pressures without balloon rupture.

As shown in FIG. 1, the hollow core 18 traverses the entire apparatus 10.
Typically the hollow core connects via a proximal ISO connector to an oxygen source such as an anesthesia circuit or the like. It is designed to allow the patient to be ventilated upon inflation of the balloons during the procedure, when the airway is otherwise occluded. The hollow core 18 is typically in the form of a central ventilating tube which is necessarily strong to prevent the pressure of the balloons from crushing the ventilating tube. The structure of the hollow core 18 is typically similar to a small endotracheal tube with a dilating cuff, and the tube may be reinforced, e.g. with wire, in the area of the cuff.

While standard balloon dilators typically have a very small central lumen to permit passage of a guidewire only, the balloon dilator of the present invention can have a fairly rigid (e.g. wire-reinforced) and relatively large central hollow core that can permit limited ventilation. For example, an 8.0 mm balloon dilator (i.e. having an outer diameter of 8.0 mm when inflated) can have a central ventilating lumen with a 2.0 mm inner diameter and a 3.0 mm outer diameter, while a 16mm balloon dilator can have a central ventilating core with a 4.0 mm inner diameter and a 5.5 mm outer diameter. According to the invention, the central ventilating lumen has an inner diameter from 2.0 mm to 4.0 mm.

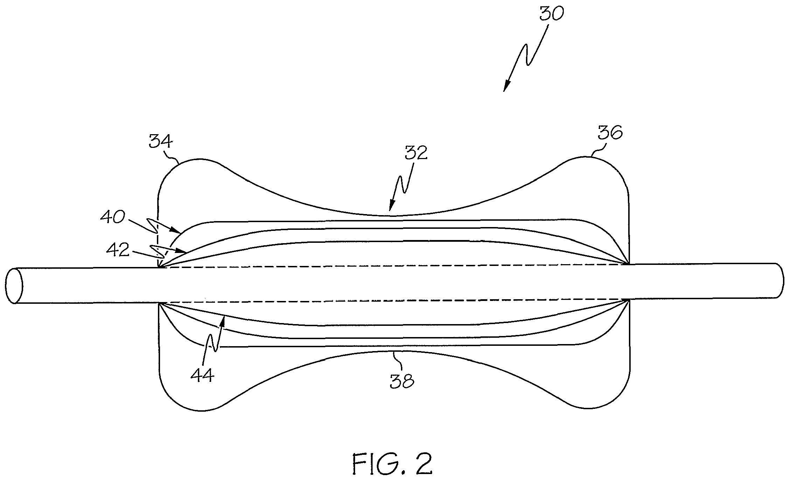

As illustrated in FIG. 2, one embodiment of the invention is a balloon dilator apparatus 30 in which the inflatable outer balloon 32 is dumbbell-shaped. This dumbbell shape typically is created by making the proximal 34 and distal 36 ends of the balloon with a decreased balloon wall thickness as compared to the central section 38 of the balloon, which has a relatively increased balloon wall thickness. Alternatively, the central section 38 can have a flexible casing or layer of plastic or the like surrounding it (not shown), thereby preventing the central section 38 from dilating as quickly as the proximal and distal ends, 34, 36, yet still permitting complete inflation of the central section 38 at the higher inflation pressures.

The dumbbell shape prevents balloon slippage by inflating at either end (i.e. on either side of the stenosis) before the central section 38 inflates, and allows the central section 38 of the outer balloon 32 to stay in position over the stenosis during inflation. During inflation, the proximal 34 and distal 36 ends of the outer balloon 32 inflate first, forming the "dumbbell" shape, thereby trapping the stenotic airway segment at the central portion 38 of the balloon 32, so that the outer balloon 32 does not slip out of position. Then, as the pressure in the balloon is increased, the central portion 38 of the balloon fully inflates at the site of the stenosis.

The present invention can provide a balloon dilator with a rated burst pressure of up to 3040 kPa (30 Atmospheres (atm)). Generally, the larger the balloon diameter, the lower the burst pressure (e.g. for comparable Blue Max^{®} angioplasty balloons, a 6.0 mm balloon has a rated burst pressure of 2026.5 kPa (20 atm), while a 14.0 mm balloon has a rated burst pressure of 810.6 kPa (8 atm), and a 20.0 mm balloon has a rated burst pressure of 314 kPa (3.1 atm). To achieve this, the present invention provides an balloon which acts as an outer "sheath" that contains a series of inner balloons with smaller individual diameters that can tolerate a higher rated burst pressure than the outer balloon.

As shown in FIG. 2, the apparatus 30 can include a plurality of inner balloons 40, 42, 44. In the embodiment shown, inner balloon 44 is contained inside inner balloon 42, which is contained inside inner balloon 40. All of the inner balloons 40, 42, 44 are contained inside outer balloon 32, and are typically separately inflatable. Such an embodiment could be used with larger diameter outer balloons, e.g. between about 10 to about 20 mm. In this embodiment, the inner balloons 40, 42, 44 can be either dumbbell shaped or a "double cone" shape as seen with most angioplasty type balloons, and are inflated sequentially if higher pressures cannot be achieved by the outer balloon. Having a balloon dilator that incorporates multiple interconnected smaller balloons can achieve the desired pressure without risking balloon rupture during inflation. In another embodiment (not shown), the inner balloons 40, 42, 44 are all contained inside the outer balloon 32 but are not contained within one another. In this embodiment the inner balloons can be interconnected so that they all inflate simultaneously, like petals of a flower around the central core, within the outer balloon.

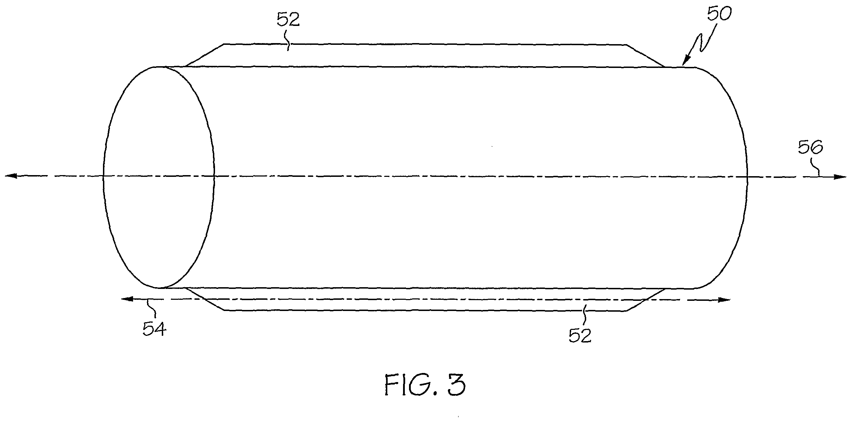

As illustrated in FIG. 3, one embodiment of the invention can include a flexible support member 50 that can fit over the apparatus, specifically fitting over the outer balloon. The flexible support member 50 is typically made of a polyurethane material and includes a central axis 56 mounted along the central axis of the apparatus. The support member is adapted to fit over the external surface of the outer balloon, and is typically substantially compliant therewith during inflation and deflation. Support member 50 also includes at least one microsurgical blade, and in FIG. 3 two surgical blades 52 are attached. Blades 52 form an effective cutting edge upon inflation of the outer balloon. Blades 52 are typically made of stainless steel, and are elongated and permanently mounted on the flexible support member 50. In use, when the support member 50 is placed over the outer balloon, the blade axis 54 is parallel to the central axis 56 of the support member 50, which is substantially parallel to the central axis of the apparatus.

Having surgical blades 52 present on the apparatus during dilation typically permits controlled cutting or lysis of any scar tissue present in the patient's airway. The blades 52 should be clearly marked so that users can avoid inadvertently cutting themselves during placement of the support member 50 over the outer balloon. In one embodiment, the blades 52 lay flat on the surface of the support member prior to use and prior to inflation of the outer balloon 12, and then when the outer balloon reaches a certain pressure upon inflation the blades 52 will typically "stand up" or otherwise protrude or expose their cutting edge atop the flexible support member 50. Once fully deployed, the exposed edge of the blade 52 typically only protrudes between about 0.2 to about 0.4 mm, and the length of the blade is typically less than the length of the outer balloon 12. Typically there are a plurality of blades which are able to work together to embed into the stenosis or scar at a substantially uniform depth. For example, three blades could be permanently mounted on the flexible support member, each of the blades being separated from the other blades so that each blade is free to move from a relatively flat position to a cutting position on the flexible support member upon inflation of the outer balloon.

In practice, the airway balloon dilation procedure is typically performed at the site of a stenosis in the airway of a patient (i.e. the larynx, trachea or bronchi). Using the apparatus shown in FIG. 15 the surgeon or clinician first inserts the apparatus 10 into the airway, then advances the apparatus within the airway until the outer balloon 12 is across the stenosis. At this point, the surgeon or clinician inflates the outer balloon 12 to cause and allow the external surface 14 of the outer balloon 12 to expand upon and dilate the stenosis. To increase dilation pressures, the inner balloon 20 is then slowly inflated. Typically the inner balloon 20 is inflated after the inflation of the outer balloon 12. Under direct visualization, the balloons are typically inflated from between about 30 to about 120 seconds. The apparatus 10 can also be threaded over a guidewire (not shown) which fits through the hollow core 18 and is positioned across the stenosis. Repeat inflation-deflation cycles can be done if airway narrowing remains after the initial attempt.

During balloon dilation, the size of the balloon is first selected by the clinician, which depends upon the size of the stenosis in the patient's airway. The balloon size is typically between about 10mm to about 40mm in length. The outer balloon is positioned over the stenosis and then each balloon is individually dilated to the desired pressure with a balloon pump, typically to between about 810.6 kPa to about 2026.5 kPa (about 8 to about 20 atmospheres). After these pressures are maintained for a predetermined period of time, typically between about 60 to about 180 seconds, the balloons are deflated and the clinician determines if repeat inflation is necessary. Repeat inflation can be safely performed if there is no obvious trauma to the airway.

While the balloon dilator of the present invention allows ventilation while inflated, the balloon dilator can also be manufactured without an inner hollow core for ventilation, but simply with a small lumen large enough to pass a guidewire. The advantage of such an embodiment (which is outside the scope of the present invention) is that the un-inflated balloon without a hollow core for ventilation is typically much "skinnier" and can pass through a very small hole (lumen) in the trachea or airway easier than a balloon dilator with a hollow core adapted to allow the patient to be ventilated therethrough.

While the present invention has been illustrated by the description of embodiments and examples thereof, it is not intended to restrict or in any way limit the scope of the appended claims to such detail. Additional advantages and modifications will be readily apparent to those skilled in the art. Accordingly, departures may be made from such details without departing from the scope of the invention.

## Claims

1. An apparatus (10; 30) for performing an airway balloon dilation procedure at the site of a stenosis in the airway of a patient, the apparatus comprising:
a central axis (16);
a hollow core (18);
an inflatable outer balloon (12; 32) having an external surface (14); and
at least one inflatable inner balloon (20; 40, 42, 44),
wherein the apparatus is insertable into the airway of a patient for movement of the balloons therein between a deflated configuration and an inflated configuration, the at least one inner balloon (20; 40, 42, 44) configured to inflate inside the outer balloon (12; 32) yet separately from the outer balloon, and having, in the inflated configuration, a smaller diameter than the outer balloon has in the inflated configuration, wherein the balloons are adapted to be individually dilated to between 810.6 kPa (8 atmospheres) to 2026.5 kPa (20 atmospheres), **characterised in that** the hollow core is adapted to allow the patient to be ventilated therethrough by having a central ventilating lumen with an inner diameter from 2.0 mm to 4.0 mm.

2. The apparatus according to claim 1, wherein the inflatable outer balloon (32) is dumbbell-shaped to hold the outer balloon in position over the stenosis.

3. The apparatus according to claim 1 or 2, comprising a plurality of inflatable inner balloons (40, 42, 44).

4. The apparatus according to claim 3, wherein there are three inner balloons (40, 42, 44) including a first inner balloon (44) contained inside a second inner balloon (42), the second inner balloon being contained inside a third inner balloon (40), all inner balloons being contained inside the outer balloon (32) and being adapted to be separately inflatable.

5. The apparatus according to claim 3, including a plurality of inflatable inner balloons (40, 42, 44), wherein all inner balloons are contained inside the outer balloon (32) and are adapted to be simultaneously inflatable within the outer balloon.

6. The apparatus according to any of claims 1 to 5, further comprising a flexible support member (50) mounted along the central axis (16) of the apparatus and on the external surface (14) of the outer balloon (12; 32), the flexible support member (50) being substantially compliant with the external surface (14) of the outer balloon (12; 32) during movement therewith, and at least one microsurgical blade (52) attached to the support member (50) and adapted to form an effective cutting edge upon inflation of the outer balloon.

7. The apparatus according to claim 6, wherein the apparatus comprises a plurality of blades (52) adapted to embed into the stenosis at a substantially uniform depth.

8. The apparatus according to claim 6 or 7, wherein the support member (50) is made of a polyurethane material and the at least one blade (52) is made of stainless steel.

9. The apparatus according to any of claims 6 to 8, wherein the at least one blade (52) includes a blade axis (54), the at least one blade (52) being elongated and mounted on the support member (50) with the blade axis (54) substantially parallel to the central axis (56) of the apparatus.

## Patentansprüche

1. Vorrichtung (10; 30) zur Durchführung eines Atemwegserweiterungsverfahrens mittels Ballon an der Stelle einer Stenose im Atemweg eines Patienten, wobei die Vorrichtung aufweist:
eine zentrale Achse (16);
einen holen Kern (18);
einen aufblasbaren äußeren Ballon (12; 32) mit einer Außenoberfläche (14); und
mindestens einen aufblasbaren inneren Ballon (20; 40, 42, 44),
wobei die Vorrichtung in den Atemweg eines Patienten einführbar ist, um die Ballone darin zwischen einem Zustand mit herausgelassener Luft und einem aufgeblasenen Zustand zu bewegen, wobei der mindestens eine innere Ballon (20; 40, 42, 44) zum Aufblasen im Inneren des äußeren Ballons (12; 32), aber getrennt von dem äußeren Ballon gestaltet ist, und im aufgeblasenen Zustand einen kleineren Durchmesser hat als der äußere Ballon im aufgeblasenen Zustand hat,
wobei die Ballone dazu ausgelegt sind, individuell auf zwischen 810, 6 kPa (8 Atmosphären) bis 2026,5 kPa (20 Atmosphären) erweitert zu werden,
**dadurch gekennzeichnet, dass** der hohle Kern dazu ausgelegt ist, zu erlauben, dass der Patient durch ihn hindurch beatmet wird, indem er ein zentrales Beatmungslumen mit einem Innendurchmesser von 2,0 mm bis 4,0 mm hat.

2. Vorrichtung nach Anspruch 1, wobei der aufblasbare äußere Ballon (32) hantelförmig ist, um den äußeren Ballon über der Stenose in Position zu halten.

3. Vorrichtung nach Anspruch 1 oder 2, eine Mehrzahl von aufblasbaren inneren Ballonen (40, 42, 44) aufweisend.

4. Vorrichtung nach Anspruch 3, bei der es drei innere Ballone (40, 42, 44) gibt, wobei ein erster innerer Ballon (44) im Inneren eines zweiten inneren Ballons (42) enthalten ist, der zweite innere Ballon im Inneren eines dritten inneren Ballons (40) enthalten ist, alle inneren Ballone im Inneren des äußeren Bal-Ions (32) enthalten sind und dazu ausgelegt sind, getrennt aufblasbar zu sein.

5. Vorrichtung nach Anspruch 3, die eine Mehrzahl aufblasbarer innerer Ballone (40, 42, 44) umfasst, wobei alle inneren Ballone im Inneren des äußeren Ballons (32) enthalten sind und dazu ausgelegt sind, gleichzeitig mit dem äußeren Ballon aufblasbar zu sein.

6. Vorrichtung nach einem der Ansprüche 1 bis 5, außerdem aufweisend ein flexibles Halteelement (50), das entlang der zentralen Achse (16) der Vorrichtung und an der Außenoberfläche (14) des äußeren Ballons (12; 32) befestigt ist, wobei das flexible Halteelement (50) mit der Außenoberfläche (14) des äußeren Ballons (12; 32) im wesentlichen konform ist, während es mit ihm bewegt wird, und mindestens eine mikrochirurgische Klinge (52), die an dem Halteelement (50) befestigt ist und dazu ausgelegt ist, beim Aufblasen des äußeren Ballons eine wirkungsvolle Schneide zu bilden.

7. Vorrichtung nach Anspruch 6, wobei die Vorrichtung eine Mehrzahl an Klingen (52) aufweist, die dazu ausgelegt sind, sich mit einer im wesentlichen gleichmäßigen Tiefe in die Stenose einzugraben.

8. Vorrichtung nach Anspruch 6 oder 7, wobei das Halteelement (50) aus einem Polyurethanmaterial hergestellt ist und die mindestens eine Klinge (52) aus rostfreiem Stahl hergestellt ist.

9. Vorrichtung nach einem der Ansprüche 6 bis 8, wobei die mindestens eine Klinge (52) eine Klingenachse (54) hat, wobei die mindestens eine Klinge (52) länglich ist und mit der Klingenachse (54) im wesentlichen parallel zu der zentralen Achse (56) der Vorrichtung an dem Halteelement (50) befestigt ist.

## Revendications

1. Appareil (10 ; 30) pour exécuter une opération de dilatation des voies aériennes au ballonnet, au site d'une sténose dans les voies aériennes d'un patient, l'appareil comprenant :
un axe central (16) ;
une partie centrale creuse (18) ;
un ballonnet externe gonflable (12 ; 32) possédant une surface externe (14) ; et
au moins un ballonnet interne gonflable (20 ; 40, 42, 44) ;
dans lequel l'appareil peut être inséré dans les voies aériennes d'un patient pour faire en sorte que les ballonnets y passent d'une configuration dégonflée à une configuration gonflée et vice versa, ledit au moins un ballonnet interne (20 ; 40, 42, 44) étant configuré pour son gonflage à l'intérieur du ballonnet externe (12 ; 32), mais de manière séparée par rapport à celui du ballonnet externe, et possédant, dans la configuration gonflée, un diamètre inférieur à celui que possède le ballonnet externe dans la configuration gonflée ;
dans lequel les ballonnets sont conçus pour une dilatation individuelle jusqu'à une valeur entre 810,6 kPa (8 atmosphères) et 2026,5 kPa (20 atmosphères), **caractérisé en ce que** la partie centrale creuse est conçue pour permettre la ventilation du patient à travers lui par le fait qu'il possède une lumière centrale de ventilation dont le diamètre interne s'élève de 2,0 mm à 4,0 mm.

2. Appareil selon la revendication 1, dans lequel le ballonnet externe gonflable (32) possède une configuration en forme d'haltère pour maintenir le ballonnet externe en position pardessus la sténose.

3. Appareil selon la revendication 1 ou 2, comprenant plusieurs ballonnets internes gonflables (40, 42, 44).

4. Appareil selon la revendication 3, dans lequel on prévoit trois ballonnets internes (40, 42, 44) englobant un premier ballonnet interne (44) contenu à l'intérieur d'un deuxième ballonnet interne (42), le deuxième ballonnet interne étant contenu à l'intérieur d'un troisième ballonnet interne (40), tous les ballonnets internes étant contenus à l'intérieur du ballonnet externe (32) et étant conçus pour pouvoir être gonflés de manière séparée.

5. Appareil selon la revendication 3, englobant plusieurs ballonnets internes gonflables (40, 42, 44), tous les ballonnets internes étant contenus à l'intérieur du ballonnet externe (32) et étant conçus pour pouvoir être gonflés de manière simultanée au sein du ballon externe.

6. Appareil selon l'une quelconque des revendications 1 à 5, comprenant en outre un élément de support flexible (50) monté le long de l'axe central (16) de l'appareil et sur la surface externe (14) du ballon externe (12 ; 32), l'élément de support flexible (50) épousant de manière substantielle la configuration de la surface externe (14) du ballonnet externe (12 ; 32) lors de leurs mouvements conjoints, et au moins une lame microchirurgicale (52) fixée à l'élément de support (50) et conçue pour former un bord tranchant efficace après le gonflage du ballon externe.

7. Appareil selon la revendication 6, dans lequel l'appareil comprend plusieurs lames (52) conçues pour s'insérer dans la sténose à une profondeur essentiellement uniforme.

8. Appareil selon la revendication 6 ou 7, dans lequel l'élément de support (50) est constitué d'une matière à base de polyuréthane et ladite au moins une lame (52) est réalisée en acier inoxydable.

9. Appareil selon l'une quelconque des revendications 6 à 8, dans lequel ladite au moins une lame (52) englobe un axe de lame (54), ladite au moins une lame (52) étant allongée et montée sur l'élément de support (50) d'une manière telle que l'axe (54) de la lame est essentiellement parallèle à l'axe central (56) de l'appareil.
